# EUROPEAN PATENT SPECIFICATION

(11) **EP 3 563 828 B1**
(45) Date of publication and mention of the grant of the patent: **15.10.2025**
(21) Application number: 17888497.9
(22) Date of filing: 27.12.2017
(51) Int. Cl.: A61K 8/44, A61K 8/02, A61K 8/34, A61K 8/39, A61K 8/86, A61Q 19/10, C11D 1/06, C11D 1/10, C11D 1/83, C11D 1/94, C11D 3/20, C11D 17/04, C11D 17/08, A61K 8/04

(54) **ARTICLE FOR SKIN CLEANSING AGENT**
ARTIKEL FÜR HAUTREINIGUNGSMITTEL
ARTICLE DESTINÉ À UN AGENT DE NETTOYAGE DE LA PEAU

(30) Priority: 28.12.2016 JP 2016255334
(43) Date of publication of application: 06.11.2019
(73) Proprietor: Kao Corporation, Chuo-ku, Tokyo 103-8210 (JP)
(72) Inventor: AOYAMA, Ryohei, Tokyo 131-8501 (JP); YASHIMA, Noboru, Tokyo 131-8501 (JP); NAGASAKI, Yuko, Tokyo 131-8501 (JP); OZAWA, Toshiaki, Tokyo 131-8501 (JP)
(74) Representative: Hoffmann Eitle
(86) International application number: PCT/JP2017/047035
(87) International publication number: WO 2018/124212

(56) References cited:
- JP-A- 2004 137 247
- JP-A- 2007 261 688
- JP-A- 2009 120 546
- JP-A- 2012 107 147
- JP-A- 2013 181 006
- JP-A- 2013 184 915
- JP-A- 2014 024 875
- US-B2- 9 132 292

## Description

### Field of the Invention

The present invention relates to an article for skin cleansing agent.

### Background of the Invention

Heretofore, it is known that a liquid cleansing composition is filled in various non-aerosol containers capable of discharging a content in a form state, to thereby apply a foam-like composition to the skin from a discharge outlet upon use and to provide, for example, a feeling upon use derived from foam. Thus, for example, as disclosed in Patent Literatures 1 and 2, there are known a pump container and a foamer dispenser including an air cylinder, a liquid cylinder, and a gas-liquid mixing part for mixing air from the air cylinder with a liquid from the liquid cylinder for foaming. The latter foamer dispenser can sufficiently secure an amount of air supplied to the gas-liquid mixing part.

Meanwhile, an attempt is also made to combine the specific container with the liquid cleansing composition to improve a desired effect. For example, Patent Literature 3 discloses a foam-generating kit which includes a foam-generating dispenser and a high concentration surfactant composition with the intention to improve detergency while generating foams having a large volume. Patent Literature 4 discloses a skin cleansing agent including a foam-discharging container having a plurality of porous membranes filled with a cleansing liquid containing a specific amount of a higher-fatty acid salt. Furthermore, Patent Literature 5 discloses a liquid cleansing composition which contains a specific amount of an anionic surfactant and a specific amount of an amine oxide type surfactant. The liquid cleansing composition is accommodated in a container which includes a foam-discharging pump mixing a content liquid with air at a volume ratio (content liquid/air) of 1/5 to 1/10 and discharging the content liquid in a foam state. These techniques improve foam quality and the like to impart a massaging effect, and improve foaming and the like under a low temperature condition.
Patent US 9 132 292 B2 discloses a foamable oil-in-water emulsion comprising: A) one or more emulsifiers, where at least one nonionic emulsifier is present, B) one or more cosurfactants, C) one or more oils, D) optionally one or more polar solubilizers, E) optionally auxiliaries and additives, F) one or more surfactants, G) water, H) optionally water-soluble substances, with the proviso that the water content of the emulsion is 70% by weight and the content of surfactant(s) (F) is from 0.01 to less than 10% by weight, based on the total emulsion.
Patent publication JP 2014 024875 A discloses a liquid detergent held in a pump foamer container, the liquid detergent containing 5% of sodium cocoyl glutamate, 11% of glycerin, 2% of polyethylene glycol 6000, 3.5% of propylene glycol and 72.75% of water.
Patent publication JP 2007 261688 A discloses a (non-aerosol type) foam discharging container for storing a liquid detergent composition, which comprises a discharge pump including a discharge opening, a mixing chamber in which a liquid detergent composition and air are mixed, and a foaming member.
Patent publication JP 2012 107147 A discloses a liquid detergent composition in a foamer container containing (A) a surfactant and (B) a polyhydric alcohol in an amount of 5 to 20% by mass.
Patent publication JP 2009 120546 A discloses a liquid detergent composition containing an N-acyl amino acid type anionic surfactant, an amphoteric surfactant and polyethylene glycol having an average molecular weight of from 1000 to 20000.
Patent publication JP 2004 137247 A discloses a liquid detergent for filling a foamer pump container, containing 3 to 30% by mass of an alkanolamine salt of an acylamino acid, 3 to 20 mass% of a polyhydric alcohol selected from the group consisting of polyethylene glycol, propylene glycol, dipropylene glycol, 1,3-butylene glycol, pentylene glycol, glycerin, diglycerin, triglycerin, and polyglycerin, and 0.1 to 1% by mass of a thickener which is an anionic polymer or a nonionic polymer.
Patent publication JP 2013 181006 A discloses a skin cleansing composition in a foamer container containing an alkyl ether carboxylic acid or salt thereof represented by the general formula (1).

(Patent Literature 1): JP-A-2005-103425
(Patent Literature 2): JP-A-2015-105120
(Patent Literature 3): JP-A-2010-18806
(Patent Literature 4): JP-A-7-252133
(Patent Literature 5): JP-A-2008-214403

The present invention relates to an article for skin cleansing agent comprising a non-aerosol-type container filled with a skin cleansing composition,
wherein:
the non-aerosol-type container comprises a gas-liquid mixing part, a foam micronizing part, and a discharge outlet, and has a mechanism for discharging a content at a volume ratio of air to the skin cleansing composition (air/composition) of 20/1 or more and 30/1 or less from the discharge outlet; and
the skin cleansing composition comprises the following components (A) to (C):
   (A) 0.5% by mass or more and 18% by mass or less, in terms of acid, of one or more anionic surfactants selected from the group consisting of an N-acylamino acid salt, and a polyoxyethylene alkyl ether carboxylic acid or a salt thereof;
   (B) 2% by mass or more and 50% by mass or less of one or more polyols selected from the group consisting of glycerin, sorbitol, propylene glycol, butylene glycol, dipropylene glycol, and polyethylene glycol having a mass average molecular weight of 20,000 or less; and
   (C) 40% by mass or more and 90% by mass or less of water;
      wherein a total content of the components (A) and (B) in the skin cleansing composition is 10% by mass or more and 60% by mass or less;
      wherein the mass average molecular weight of the polyethylene glycol is measured by gel permeation chromatography.

In this context, if a gas-liquid ratio is increased so that more air can be introduced with respect to a liquid cleansing agent as the content in the gas-liquid mixing part included in the non-aerosol-type container, the generation of foams having a high air ratio is expected, but good foam quality is not necessarily imparted. This still requires further improvement.

That is, the present disclosure relates to an article for skin cleansing agent which can generate foams having a high air ratio and achieve good foam quality.

Then, the present inventors made various investigations, and used specific amounts of a specific anionic surfactant and polyol, in combination with a specific non-aerosol-type container having a mechanism for discharging a content at a volume ratio of air to a skin cleansing composition (air/composition) of 20/1 or more and 30/1 or less from a discharge outlet in the presence of a specific amount of water, to find an article for skin cleansing agent which can form foams having good foam quality while having a high air ratio upon use.

The article for skin cleansing agent of the present disclosure can attain a foam-like skin cleansing agent which is fine and elastic, provides good foam quality while having a high air ratio when the skin cleansing agent is discharged from the non-aerosol-type container upon use, and can provide comfortable feeling upon use while exhibiting high detergency.

### Brief Description of Drawings

[Figure 1] Figure 1 is a graph showing the relationship between a time period during which shear is applied and a shear rate when a measuring device (MCR 502 (Modular Compact Rheometer, Anton Paar, Measurement Cell: P-PTD200, Measurement System: PP50) is used in measurement of a viscosity coefficient in Examples.
[Figure 2] Figure 2 is a schematic diagram showing foams observed by using a foam diameter analyzer (DFA 100, Foam Analysis, KRUSS).

### Detailed Description of the invention

The article for skin cleansing agent of the present disclosure is an article for skin cleansing agent containing a non-aerosol-type container (X) to be described later filled with a skin cleansing composition (Y).

The non-aerosol-type container (X) used in the present invention contains a gas-liquid mixing part, a foam micronizing part, and a discharge outlet, and has a mechanism for discharging a content at a volume ratio of air to the skin cleansing composition (Y) to be described later (air/composition) of 20/1 or more and 30/1 or less from the discharge outlet. A non-aerosol-type container conventionally and frequently used is merely a container having a mechanism for mixing air with a content liquid at a volume ratio (air/content liquid) of 5/1 to 13/1, as described also in, for example, JP-A-2008-214403. Meanwhile, the non-aerosol-type container (X) used in the present invention is a container which has a mechanism for deriving and discharging air at a volume ratio of the air to the skin cleansing composition (Y) (air/composition) of 20/1 or more and 30/1 or less, which is higher ratio than that of the liquid solution, to allow foams having a high air ratio to be discharged.

Herein, "good foam quality" semantically includes fineness (density) and high elasticity.

The non-aerosol-type container (X) is a normal-pressure container which includes the gas-liquid mixing part, the foam micronizing part, and the discharge outlet, and eliminates the need for a propellant such as a compressed gas. The gas-liquid mixing part is an area into which air transported from the outside or inside of the container (X) and the liquid skin cleansing composition (Y) filled in the container (X) as a content by storing in the main body or a member such as a tank flow and are mixed. The foam micronizing part is an area or a member which contains a flow path having a specific shape or a porous member and the like provided in the flow path to the discharge outlet, and makes the foam obtained by mixing the air and the skin cleansing composition (Y) fine. The discharge outlet is a member which discharges foams generated by mixing air with the skin cleansing composition (Y) and made fine by the foam micronizing part to the outside.

That is, the gas-liquid mixing part, the foam micronizing part, and the discharge outlet included in the container (X) are directly or indirectly linked in this order via a member such as a tube or a nozzle, if needed. According to manual or electric power to be added, the skin cleansing composition (Y) filled in the main body of the non-aerosol-type container (X) and a member such as a tank as a liquid goes from the gas-liquid mixing part through the foam micronizing part, and subsequently from the foam micronizing part through the discharge outlet to become the fine foam-like skin cleansing composition (Y) containing therein air, which is discharged from the discharge outlet to the outside, and can applied to the skin.

From the viewpoint that the non-aerosol-type container (X) used in the present disclosure secures good foam quality while discharging foams at a high air ratio, in the mechanism for discharging the content from the discharge outlet included in the container (X), the volume ratio of the air to the skin cleansing composition (Y) (air/composition) is 20/1 or more and 30/1 or less. In the mechanism for discharging the content from the discharge outlet included in the non-aerosol-type container (X), from the viewpoints of achieving the easiness of operation in the container, and reductions in the weight and size of the container, and of securing good foam quality, the volume ratio of the air to the skin cleansing composition (Y) (air/composition) is 20/1 or more and 30/1 or less. In the mechanism for discharging the content from the discharge outlet included in the non-aerosol-type container (X), the volume ratio of the air to the skin cleansing composition (Y) (air/composition) is preferably from 20/1 to 26/1.

The volume ratio of the air to the skin cleansing composition (Y) (air/composition) means a value at 25°C.

Examples of the mechanism for discharging the content from the discharge outlet at the above-mentioned volume ratio of the air to the skin cleansing composition (air/composition) include a mechanism including a gas-liquid mixing part in which the air and the skin cleansing composition (Y) can be mixed at the above-mentioned volume ratio (air/composition).

Specific form of the non-aerosol-type container (X) may have a mechanism for discharging the air and the skin cleansing composition at the above-mentioned mass ratio from the gas-liquid mixing part to the foam micronizing part via a member such as a tube or a nozzle, if needed, causing the air and the skin cleansing composition to go through the foam micronizing part, and then discharging the content from the discharge outlet to the outside. Examples of the forms of non-aerosol-type container (X) include a squeeze type container, a manual pump type container, and an electric pump type container.

The squeeze type container is also referred to as a squeezing container. By manually adding a pressing force to a barrel part located in the deformable container body from the outside, that is, by squeeze-deforming the barrel part, the air pressure-fed from the inside of a head space is mixed with the liquid as the content in the gas-liquid mixing part, to form foams, and the foams are then made fine in the foam micronizing part. The fine foams are discharged from the discharge outlet. Specific examples thereof include a container described in JP-A-7-215353.

The pump type container is also referred to as a pump container. By pressing a pump head included in a foam discharger including a discharge outlet, air pressure-fed from the inside or outside of the container is mixed with a liquid as a content in a gas-liquid mixing part to form foams. The foams are made fine in a foam micronizing part, and the fine foams are discharged from the discharge outlet. That is, the pump type container includes an air cylinder for introducing air from the inside or outside of the container and a liquid cylinder serving as a flow path for the liquid as the content, and has a mechanism for sliding a piston in each of the cylinders. By pressing the pump head, the pistons are driven, and the air and the liquid as the content are delivered to the gas-liquid mixing part, to mix the air with the liquid in the gas-liquid mixing part. Then, foams are formed in the foam micronizing part, and discharged from the discharge outlet. The pump type container is broadly divided into a so-called manual pump type container in which a pump head is manually pressed and a so-called electric pump type container in which a pump head is electrically pressed and air and a content are electrically transported between members, and both the manual pump type container and the electric pump type container can be used. Specific examples thereof include a manual pump type container described in JP-A-2009-202122, and electric pump type containers described in JP-A-2006-212086 and JP-A-2013-212244.

Among the specific forms of the non-aerosol-type container (X), from the viewpoints of stably securing a high air ratio, of securing the formation of foams having good quality, and of the easiness of operation in the container, the manual pump type container or the electric pump type container is preferred.

Furthermore, from the viewpoint of satisfactorily forming a foam-like composition when the content goes through the discharge outlet, and from the viewpoint that the formed foams have good foam quality to improve a feeling upon use and the like, the non-aerosol-type container (X) used in the present invention preferably includes a porous member having a mesh or a plurality of small holes in the foam micronizing part. The opening of the mesh is preferably 16 to 180 µm (#510 to #90). In lights of the usability of the container and the foam quality to be obtained, the opening of the mesh is more preferably 40 to 80 µm (#330 to #180).

The skin cleansing composition (Y) used in the present invention contains the following components (A) to (C).

The skin cleansing composition (Y) contains one or more anionic surfactants selected from the group consisting of an N-acylamino acid salt, and a polyoxyethylene alkyl ether carboxylic acid or a salt thereof in an amount of 0.5% by mass or more and 18% by mass or less in terms of acid, as the component (A). This can provide good foam quality while exhibiting excellent foaming ability. In the present invention, the content of the component (A) in terms of acid means the content of the component (A) as acid.

From the viewpoints of securing high detergency, and of keeping good discharge properties, an acyl group of the N-acylamino acid salt is preferably derived from a saturated or unsaturated and linear or branched fatty acid having 4-30 carbon atoms, more preferably derived from a saturated or unsaturated and linear or branched fatty acid having 6-26 carbon atoms, even more preferably derived from a saturated or unsaturated and linear or branched fatty acid having 8-24 carbon atoms.

Examples of the fatty acid include one or more selected from the group consisting of caprylic acid, capric acid, lauric acid, myristic acid, palmitic acid, stearic acid, oleic acid and the like. Among these fatty acids, from the viewpoint of improving foam quality and storage stability, one or more selected from the group consisting of lauric acid, myristic acid, palmitic acid, and oleic acid are preferred, and lauric acid is more preferred. The acyl group of the N-acylamino acid may be derived from a mixed fatty acid of the fatty acids above, for example, may be obtained by using coconut oil, palm kernel oil and the like as raw materials. Among these, the acyl group is preferably derived from coconut oil fatty acid or palm kernel fatty acid, more preferably derived from coconut oil fatty acid.

The salt of the N-acylamino acid salt is preferably an alkali metal such as sodium or potassium; ammonium; ammonium derived from alkanolamine such as monoethanolamine, diethanolamine, or triethanolamine; and cation derived from basic amino acid such as arginine or lysine.

The N-acylamino acid salt may be any of an L isomer, a D isomer, and a racemic isomer.

The polyoxyethylene alkyl ether carboxylic acid or the salt thereof is specifically represented by the following formula (1). 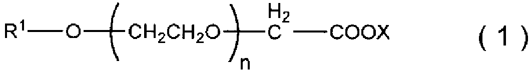 (In the formula (1), R¹ represents an alkyl group or alkenyl group having 10-18 carbon atoms; n represents the number of 0.5 to 10 on average; and X represents a hydrogen atom, an alkali metal, an alkaline-earth metal, ammonium, or organic ammonium.)

In the formula (1), R¹ is preferably an alkyl group having 12-16 carbon atoms. An average molar number n of addition of ethylene oxide is particularly preferably from 1 to 6.

X is preferably an alkali metal such as sodium or potassium; ammonium; ammonium derived from alkanolamine such as monoethanolamine, diethanolamine, or triethanolamine; and cation derived from basic amino acid such as arginine or lysine.

For example, a polyoxyethylene alkyl ether carboxylic acid or a salt thereof described in JP-A-2013-053092 is also preferred.

From the viewpoint of securing good foam quality, the content of the component (A) is 0.5% by mass or more, preferably 1% by mass or more, more preferably 2% by mass or more in terms of acid in the skin cleansing composition (Y) and from the viewpoint of keeping good discharge properties, the content of the component (A) is 18% by mass or less, preferably 15% by mass or less, more preferably 12% by mass or less, even more preferably 10% by mass or less in terms of acid in the skin cleansing composition (Y). The content of the component (A) is 0.5% by mass or more and 18% by mass or less, preferably from 0.5 to 15% by mass, more preferably from 1 to 12% by mass, even more preferably from 2 to 10% by mass in terms of acid in the skin cleansing composition (Y).

The skin cleansing composition (Y) contains 2% by mass or more and 50% by mass or less of one or more polyols selected from the group consisting of glycerin, sorbitol, propylene glycol, butylene glycol, dipropylene glycol, and polyethylene glycol having a mass average molecular weight of 20,000 or less (measured by gel permeation chromatography) as the component (B). The component (B) imparts an appropriate viscosity to the skin cleansing composition (Y) filled in the non-aerosol-type container (X), in combination with the component (A), whereby good foam quality can be provided while good discharge properties are secured.

The mass average molecular weight of the polyethylene glycol means a value measured by GPC (gel permeation chromatography). From the viewpoint of imparting an appropriate viscosity to secure good discharge properties, the mass average molecular weight of the polyethylene glycol used as the component (B) is 20,000 or less, preferably 15,000 or less, more preferably 10,000 or less. From the viewpoint of obtaining good foam quality, the mass average molecular weight of the polyethylene glycol is preferably 200 or more, more preferably 400 or more.

From the viewpoint of securing good foam quality, the content of the component (B) is 2% by mass or more, preferably 3% by mass or more, more preferably 5% by mass or more, even more preferably 8% by mass or more in the skin cleansing composition (Y) and from the viewpoint of keeping good discharge properties, the content of the component (B) is 50% by mass or less, preferably 45% by mass or less, more preferably 40% by mass or less, even more preferably 35% by mass or less in the skin cleansing composition (Y). The content of the component (B) is 2% by mass or more and 50% by mass or less, preferably from 3 to 45% by mass, more preferably from 5 to 40% by mass, even more preferably from 8 to 35% by mass in the skin cleansing composition (Y).

Among these, from the viewpoint of improving an initial foam elastic force and the density of foams, the content of the component (B) is more preferably 9% by mass or more, even more preferably 10% by mass or more; and more preferably 20% by mass or less, even more preferably 15% by mass or less in the skin cleansing composition (Y). The content of the component (B) is more preferably from 9 to 20% by mass, and still more preferably from 10 to 15% by mass in the skin cleansing composition (Y).

From the viewpoint of imparting good foam quality while securing a high air ratio, the total content of the components (A) and (B) is 10% by mass or more, more preferably 15% by mass or more, even more preferably 17% by mass or more, even more preferably 18% by mass or more in the skin cleansing composition (Y) and the total content of the components (A) and (B) is 60% by mass or less, more preferably 55% by mass or less, even more preferably 50% by mass or less, even more preferably 45 % by mass or less in the skin cleansing composition (Y). The total content of the components (A) and (B) is 10 to 60% by mass, more preferably from 15 to 55% by mass, even more preferably from 15 to 50% by mass, even more preferably from 17 to 45% by mass, even more preferably from 18 to 45% by mass in the skin cleansing composition (Y).

The skin cleansing composition (Y) contains 40% by mass or more and 90% by mass or less of water as the component (C). Thereby, each of the components is satisfactorily dispersed or dissolved, which improves foaming ability while securing an appropriate viscosity. The water as the component (C) in the present invention means total moisture contained in the skin cleansing composition (Y), which contains not only purified water or ion-exchanged water and the like blended with the skin cleansing composition (Y) but also moisture contained in each of the blended components.

From the viewpoint of satisfactorily dispersing or dissolving each of the components, the content of the component (C) is 40% by mass or more, preferably 45% by mass or more, more preferably 50% by mass or more in the skin cleansing composition (Y) and from the viewpoint of keeping an appropriate viscosity to secure a high air ratio, the content of the component (C) is 90% by mass or less, preferably 85% by mass or less, more preferably 80 % by mass or less in the skin cleansing composition (Y). The content of the component (C) is 40% by mass or more and 90% by mass or less, preferably from 45 to 85% by mass, more preferably from 50 to 85% by mass, still more preferably from 50 to 80% by mass in the skin cleansing composition (Y).

From the viewpoint of securing good foaming ability in combination with the component (A), the skin cleansing composition (Y) used in the present invention can contain a fatty acid salt. Specific examples of the fatty acid salt include a fatty acid salt having a linear or branched alkyl group or alkenyl group having 9-21 carbon atoms. More specific examples thereof include salts of lauric acid, myristic acid, palmitic acid, stearic acid, isostearic acid, oleic acid, arachic acid, and behenic acid. Preferred examples of the salts include an alkali metal such as sodium or potassium; ammonium; ammonium derived from alkanolamine such as monoethanolamine, diethanolamine, or triethanolamine; and cation derived from basic amino acid such as arginine or lysine.

Among these, from the viewpoint of securing good foam quality, a fatty acid salt having a linear or branched alkyl group or alkenyl group having 9-19 carbon atoms is preferred; a fatty acid salt having a linear or branched alkyl group or alkenyl group having 11-19 carbon atoms is more preferred; and a fatty acid salt having a linear or branched alkyl group or alkenyl group having 11-17 carbon atoms is even more preferred.

From the viewpoint of effectively improving foaming ability, the content of the fatty acid salt is preferably 0.5% by mass or more, more preferably 1% by mass or more, even more preferably 2% by mass or more in terms of acid in the skin cleansing composition (Y). From the viewpoint of securing good foam quality, the content of the fatty acid salt is preferably 10% by mass or less, more preferably 9% by mass or less, even more preferably 8% by mass or less in terms of acid in the skin cleansing composition (Y). The content of the fatty acid salt is preferably 0.5 to 10% by mass in terms of acid in the skin cleansing composition (Y), more preferably 1 to 9% by mass, and still more preferably 2 to 8% by mass. In the present invention, the content of the fatty acid salt in terms of acid means the content of the fatty acid salt as acid.

From the viewpoint of securing good foaming ability and discharge properties, the skin cleansing composition (Y) used in the present invention can contain an amphoteric surfactant (D). Specific examples of the component (D) include betaine-based surfactants such as imidazoline-based betaine, alkyl dimethyl aminoacetic acid betaine, fatty acid amide propyl betaine, and sulfobetaine; and amine oxide type surfactants such as alkyl dimethyl amine oxide. More specific examples of the component (D) include 2-alkyl-N-carboxymethyl-N-hydroxyethylimidazolinium betaine, lauryl dimethylaminoacetic acid betaine, lauric acid amidopropyl betaine, lauryl hydroxysulfobetaine, and lauryl dimethylamine oxide.

Among these, from the viewpoint of keeping good foaming ability and detergency, one or more selected from the group consisting of an amide betaine-based surfactant and a sulfobetaine-based surfactant are preferred, and an amide betaine-based surfactant is more preferred. Specifically, at least one selected from the group consisting of lauric acid amidopropyl betaine and lauryl hydroxysulfobetaine is preferred.

From the viewpoint of securing good foam quality while improving foaming ability in combination with the component (A), the content of the component (D) is preferably 0.1% by mass or more, more preferably 0.3% by mass or more, even more preferably 0.5% by mass or more in the skin cleansing composition (Y). From the viewpoint of securing good discharge properties, the content of the amphoteric surfactant is preferably 5% by mass or less, more preferably 4% by mass or less, even more preferably 3% by mass or less in the skin cleansing composition (Y). The content of the amphoteric surfactant is preferably from 0.1 to 5% by mass, more preferably from 0.3 to 4% by mass, even more preferably from 0.5 to 3% by mass in the skin cleansing composition (Y).

Among these, from the viewpoint of further improving an initial foam elastic force and the density of foams, the content of the component (D) is more preferably 0.8% by mass or more, even more preferably 1.0% by mass or more in the skin cleansing composition (Y). The content of the component (D) is more preferably less than 2.0% by mass, even more preferably 1.9% by mass or less in the skin cleansing composition (Y). The content of the component (D) is more preferably 0.8% by mass or more and less than 2.0% by mass, even more preferably from 1.0 to 1.9% by mass in the skin cleansing composition (Y).

In the present invention, anionic surfactants other than the component (A) and the fatty acid salt can be appropriately contained. From the viewpoint of improving an initial foam elastic force and the density of foams, the mass ratio of the total content of the anionic surfactants, i.e., the total content of the anionic surfactants containing the component (A) to the content of the component (D) (anionic surfactant/(D)) is preferably more than 5, more preferably 5.5 or more, even more preferably 6.0 or more. The mass ratio is preferably 100 or less, more preferably 50 or less, even more preferably 20 or less, even more preferably 10 or less. The mass ratio of the total content of the anionic surfactants containing the component (A) to the content of the component (D) (anionic surfactant/(D)) is preferably more than 5 and 100 or less, more preferably from 5.5 to 50, even more preferably from 6.0 to 20 or more, even more preferably from 6.0 to 10.

From the viewpoint of satisfactorily dispersing or dissolving each of the components while securing an appropriate viscosity, to secure good foaming ability and detergency, the skin cleansing composition (Y) used in the present invention may contain a non-ionic surfactant (E). Specific examples of the component (E) include polyethylene glycol type non-ion surfactants such as polyoxyethylene sorbitan fatty acid ester, polyoxyethylene sorbit fatty acid ester, polyoxyethylene glycerin fatty acid ester, polyoxyethylene fatty acid ester, polyoxyethylene alkyl ether, polyoxy ethylene polyoxypropylene glycol, polyoxyethylene alkyl phenyl ether, and polyoxyalkylene (hardened) castor oil; sucrose fatty acid ester; polyglycerol alkyl ether; polyglycerol fatty acid ester; sorbitan fatty acid ester; alkyl glyceryl ether; alkyl glycoside; and fatty acid alkanol amide.

Among these, from the viewpoint of keeping good foaming ability and detergency, the non-ionic surfactant preferably contains an alkyl group or alkenyl group having carbon atoms of 8 or more and 20 or less as a hydrophobic portion. More specifically, at least one selected from the group consisting of alkyl glucoside, polyoxyethylene sorbitan fatty acid ester, and polyoxyethylene alkyl ether is preferred.

From the viewpoint of satisfactorily dispersing or dissolving each of the components, the content of the component (E) is preferably 0.1% by mass or more, more preferably 0.3% by mass or more, even more preferably 0.5% by mass or more in the skin cleansing composition (Y). From the viewpoint of suppressing an unnecessary increase in viscosity to secure an appropriate viscosity, the content of the component (E) is preferably 5% by mass or less, more preferably 4% by mass or less, even more preferably 3% by mass or less in the skin cleansing composition (Y). The content of the component (E) is preferably from 0.1 to 5% by mass, more preferably from 0.3 to 4% by mass, even more preferably from 0.5 to 3% by mass in the skin cleansing composition (Y).

When the dispersibility or solubility of each of the components is good even if the component (E) is not contained, that is, when poor dispersion and precipitation do not occur even if the component (E) is not daringly added, the content of the component (E) can be reduced. Specifically, from the viewpoint of improving an initial foam elastic force and the density of foams, the content of the component (E) in this case is preferably 0.5% by mass or less, more preferably 0.1% by mass or less, even more preferably 0.05% by mass or less in the skin cleansing composition (Y). The content of the component (E) is preferably 0.001% by mass or more, more preferably 0.005% by mass or more, even more preferably 0.01% by mass or more in the skin cleansing composition (Y). The content of the component (E) when the dispersibility or solubility of each of the components is good even if the component (E) is not contained is preferably from 0.001 to 0.5% by mass, more preferably from 0.005 to 0.1% by mass, even more preferably from 0.01 to 0.05% by mass in the skin cleansing composition (Y).

From the viewpoints of satisfactorily keeping discharge properties from the container to secure excellent foam quality, and of suppressing an unnecessary increase in viscosity to secure a high air ratio, the skin cleansing composition (Y) used in the present invention preferably restricts the inclusion of a thickener. Examples of the thickener include one or more selected from the group consisting of sodium alginate, carboxymethyl cellulose or salts thereof, carrageenan, xanthan gum, sodium polyacrylate, hydroxyethyl cellulose, hydroxypropyl cellulose, pectin, gum tragacanth, gum arabic, guar gum, gum karaya, locust bean gum, gellan gum, tamarind gum, psyllium seed gum, polyvinyl alcohol, sodium chondroitin sulfate, polyethylene glycol having a mass average molecular weight of more than 20,000, a methoxyethylene-maleic anhydride copolymer, a cationic polymer, and an ampholytic polymer.

The content of the thickener may fluctuate depending on the use of the detergent and the kind of the substrate, but from the viewpoint of securing good discharge properties from the container and a high air ratio, the content of the thickener is preferably 5% by mass or less, more preferably 3% by mass or less, even more preferably 2% by mass or less in the skin cleansing composition (Y). From the viewpoint of imparting an appropriate viscosity to secure good foam quality and discharge properties, the content of the thickener is preferably 0.01% by mass or more, more preferably 0.05% by mass or more, even more preferably 0.1% by mass or more. Alternatively, the skin cleansing composition (Y) may not contain the thickener.

From the viewpoint of securing good discharge properties from the container, the skin cleansing composition (Y) used in the present invention preferably restricts the inclusion of a powder or particulate matter such as a scrub agent. Even if the powder or particulate matter is mixed with water, the powder or particulate matter is present as particles, and has a particle length (the maximum length in the case of irregularly-shaped particles) of 2 µm or more. Examples thereof include synthetic polymers such as polyethylene, polypropylene, polyamide, polyethylene terephthalate, polystyrene, polyurethane or crosslinked products thereof, sodium poly(meth)acrylate, poly(meth)acrylic acid ester or crosslinked products thereof, and rubbers such as ethylene rubber, propylene rubber, styrene-butadiene rubber, butadiene rubber, and silicone rubber, or crosslinked products thereof; natural polymers or derivatives thereof such as celluloses or derivatives thereof, chitosan or derivatives thereof, corn starch, and fruit shells, bentonite, talc, mica, kaolin, sepiolite, silica, calcium carbonate, titanium oxide, silicic acid anhydride, calcium hydroxyapatite, and mother of pearl.

The particle length means a value obtained by a laser diffraction scattering method.

From the viewpoint of securing good discharge properties from the container, the content of the powder or particulate matter is preferably 0.5% by mass or less, more preferably 0.4% by mass or less, even more preferably 0.3 % by mass or less in the skin cleansing composition (Y). The content of the powder or particulate matter is preferably more than 0% by mass. Alternatively, it is preferable that the skin cleansing composition (Y) does not contain the powder or the particulate matter. The content of the powder or particulate matter is preferably from 0 to 0.5% by mass, more preferably 0 to 0.4% by mass, even more preferably 0 to 0.3% by mass in the skin cleansing composition (Y). Alternatively, it is preferable that the skin cleansing composition (Y) does not contain the powder or the particulate matter.

From the viewpoint of improving good foam quality, and from the viewpoints of the shape retention property of foams and foam stability, the skin cleansing composition (Y) used in the present invention can contain a fine powder having a particle length of less than 2 µ. From the viewpoint of gentleness to the skin, the fine powder is preferably one or more selected from the group consisting of celluloses or derivatives thereof, chitosan or derivatives thereof, bentonite, talc, silica, and calcium carbonate, more preferably bentonite and cellulose. The particle length thereof (the maximum length in the case of irregularly-shaped particles) is preferably less than 2 µm, more preferably 1 µm or less, even more preferably 0.5 µm or less. The content of the fine powder is preferably 5% by mass or less, more preferably 2% by mass or less. Preferably, the skin cleansing composition (Y) does not contain the fine powder. Alternatively, the skin cleansing composition (Y) can contain 0.1% by mass or more of the fine powder.

From the viewpoints of securing good foam quality, and of preventing the generation of precipitates during storage, the skin cleansing composition (Y) used in the present invention preferably restricts the inclusion of ethanol. The content of the ethanol is preferably 5% by mass or less, more preferably 3% by mass or less, even preferably 1% by mass or less, even more preferably 0.1% by mass or less in the skin cleansing composition (Y). The content of the ethanol is preferably more than 0% by mass, or 0.001% by mass or more. Alternatively, it is preferable that the skin cleansing composition (Y) does not contain ethanol.

The skin cleansing composition (Y) used in the present invention can appropriately contain, in addition to the above-mentioned components, components conventionally used for the skin cleansing agent, for example, a surfactant other than the above-mentioned components, a humectant, an oil component, a disinfecting agent, an antiseptic agent, an antiperspirant, an antioxidant, an anti-inflammatory, an anti-ultraviolet agent, a cooling agent, a perfume, and a dye. The uses of the components are not limited to the original uses, and the components can be used for other uses depending on the purpose. For example, the antiperspirant can be used as the perfume.

From the viewpoint of securing good foam quality and discharge properties, the viscosity at 25°C of the skin cleansing composition (Y) is preferably 2 mPa·s or more, more preferably 3 mPa·s or more, even more preferably 5 mPa·s or more. The viscosity at 25°C of the skin cleansing composition (Y) is preferably 20 mPa·s or less, more preferably 18 mPa·s or less, even more preferably 15 mPa·s or less. The viscosity at 25°C of the skin cleansing composition (Y) is preferably from 2 to 20 mPa·s, more preferably from 3 to 18 mPa·s, even more preferably from 5 to 15 mPa·s.

The viscosity of the skin cleansing composition (Y) is a value measured as a liquid viscosity (mPa·s) at 25°C by using a helipath type viscometer (B type viscometer, rotor BM No. 1, number of rotations: 60 rpm/1 minute).

In the article for skin cleansing agent of the present invention, from the viewpoint of appropriate viscosity and of forming foams having fine foam quality, the viscosity coefficient (Pa·s) of the skin cleansing composition (Y) discharged from the non-aerosol-type container (X) is a value at an elapse of 5 seconds after a shear force is applied to the discharged skin cleansing composition (Y) and a shear rate of 1,000 (1/s) is reached. The viscosity coefficient (Pa·s) of the skin cleansing composition (Y) is preferably 6 × 10⁻² Pa·s or more, more preferably 7 × 10⁻² Pa·s or more, even more preferably 9 × 10⁻² Pa·s.

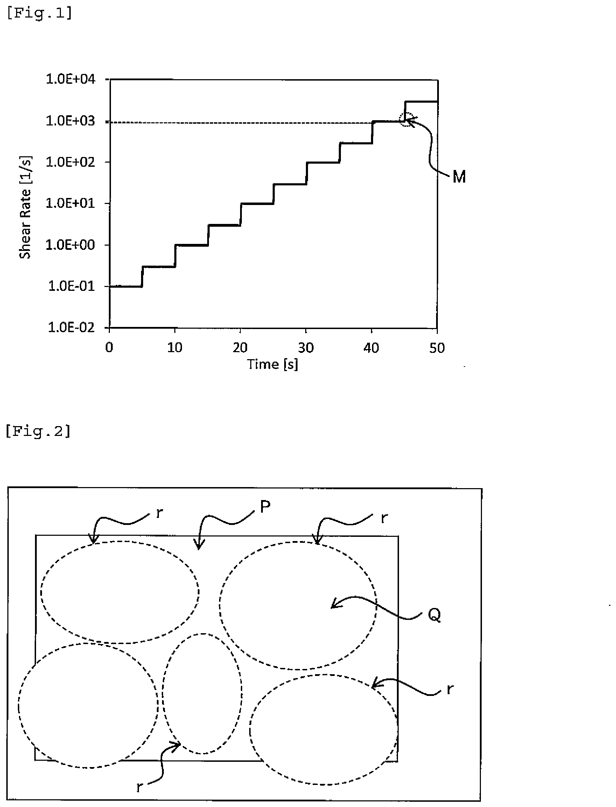

The phrase "at an elapse of 5 seconds after a shear force is applied to the discharged skin cleansing composition (Y) and a shear rate of 1,000 (1/s) is reached" specifically means a measurement point M shown in a graph of Figure 1, for example.

When the viscosity coefficient of the skin cleansing composition (Y) discharged from a non-aerosol-type container having a mechanism for discharging the content from the discharge outlet at a volume ratio (air/composition) of 13/1 is taken as a standard (100%), the increasing rate (%) of the viscosity coefficient of the skin cleansing composition (Y) discharged from the non-aerosol-type container (X) is preferably 30% or more, more preferably 40% or more, even more preferably 50% or more.

The viscosity coefficient (Pa·s) of the skin cleansing composition (Y) discharged from the non-aerosol-type container (X) is a value measured under conditions of Gap: 0.5 mm and temperature: 30°C by using a measuring device (MCR 502, Modular Compact Rheometer, manufactured by Anton Paar, Measurement Cell: P-PTD200, Measurement System: PP50).

From the viewpoints of providing good foam quality and discharge properties and of causing high foaming ability to effectively improve detergency, the expansion ratio of the skin cleansing composition (Y) is 15 times or more, preferably 17 times or more, more preferably 20 times or more at an elapse of 5 seconds after the skin cleansing composition (Y) is discharged from the non-aerosol-type container (X). The expansion ratio of the skin cleansing composition (Y) is 38 times or less, preferably 30 times or less, more preferably 29 times or less, even more preferably 26 times or less. The expansion ratio of the skin cleansing composition (Y) is 15 times or more and 38 times or less, preferably from 17 times to 30 times, more preferably from 20 times to 29 times, even more preferably from 20 times to 26 times at an elapse of 5 seconds after the skin cleansing composition (Y) is discharged from the discharge outlet.

The expansion ratio of the skin cleansing composition (Y) is a value of the volume ratio of the volume of the foam-like skin cleansing composition (Y) at an elapse of 5 seconds after the skin cleansing composition (Y) is discharged from the non-aerosol-type container (X) at 25°C to the volume of the liquid skin cleansing composition (Y) (foam-like volume/liquid volume).

In the article for skin cleansing agent of the present invention, from the viewpoint of forming foams which are fine and elastic and provide good foam quality, the number of foams of the skin cleansing composition (Y) discharged from the non-aerosol-type container (X) is preferably 500 to 10,000 in an observation region (1,280 × 1,240 pixels) according to image processing, more preferably 1,000 to 8,000, and still more preferably 1,400 to 6,000.

In the article for skin cleansing agent of the present invention, the number of foams of the skin cleansing composition (Y) discharged from the non-aerosol-type container (X) can be increased by increasing the expansion ratio. When the number of foams of the skin cleansing composition (Y) discharged from a non-aerosol-type container in which the volume ratio of the air to the skin cleansing composition (Y) (air/composition) is 13/1 is taken as a standard (100%), the increasing rate of the number of foams of the skin cleansing composition (Y) discharged from the non-aerosol-type container (X) is preferably 5% or more, more preferably 10% or more, even more preferably 15% or more. From the viewpoint of the discharge properties of the non-aerosol-type container, the increasing rate of the number of foams is preferably 90% or less, more preferably 80% or less, even more preferably 70% or less.

From the viewpoint of forming foams which are fine and elastic and provide good foam quality, the total length of the foam interface of the skin cleansing composition (Y) discharged from the non-aerosol-type container (X) is preferably from 3 × 10⁵ to 20 × 10⁵ µm, more preferably from 4 × 10⁵ to 15 × 10⁵ µm, more preferably from 5 × 10⁵ to 13 × 10⁵ µm in an observation region (1,280 × 1,240 pixels) according to image processing.

Herein, the total length of the foam interface means the total of the lengths of interfaces r between foams Q and the skin cleansing composition P confirmed in the observation region, i.e., a value obtained by summing the full lengths of circumferences r of the foams B, as shown in a schematic diagram of Figure 2.

In the article for skin cleansing agent of the present invention, the total length of the foam interface of the skin cleansing composition (Y) discharged from the non-aerosol-type container (X) can be increased by increasing the expansion ratio. When the total length of the foam interface of the skin cleansing composition (Y) discharged from a non-aerosol-type container in which the volume ratio of the air to the skin cleansing composition (Y) (air/composition) is 13/1 is taken as a standard (100%), the increasing rate of the total length of the foam interface of the skin cleansing composition (Y) discharged from the non-aerosol-type container (X) is preferably 3% or more, more preferably 5% or more, even more preferably 7% or more. From the viewpoint of the discharge properties of the non-aerosol-type container, the increasing rate of the total length of the foam interface is preferably 60% or less, more preferably 50% or less, even more preferably 40% or less.

The number of foams and the total length of the foam interface of the skin cleansing composition (Y) discharged from the non-aerosol-type container (X) are values obtained by using a foam diameter analyzer (DFA 100, Foam Analysis, manufactured by KRUSS) as a measuring device, placing foams discharged from the discharge outlet of the container on a plate, and observing and measuring foams present in an observation region (1,280 × 1,240 pixels) according to image processing.

From the viewpoints of securing good discharge properties, and of keeping low stimulation to the skin, the pH at 25°C of the skin cleansing composition (Y) is preferably 5 or more, more preferably 6 or more. The pH at 25°C of the skin cleansing composition (Y) is preferably 11 or less, more preferably 10 or less.

When the article for skin cleansing agent of the present invention is used, the skin cleansing composition (Y) may be discharged in a foam state from the discharge outlet of the non-aerosol-type container (X), and directly applied to the skin of the face, hand, or body and the like without dilution with water, followed by washing the skin and rinsing the skin with water. The high expansion ratio exhibits excellent detergency, and provides fine and elastic foams with good quality, whereby excellent feeling upon use can be realized.

### Examples

Hereinafter, the present invention will be described more specifically by way of Examples. Contents of components are represented by % by mass unless otherwise indicated in Tables.

### [Production Example: Preparation of Skin Cleansing composition]

### (Composition 1)

All components other than potassium hydroxide were mixed, and heated to 70°C, followed by uniformizing. Then, potassium hydroxide was added to the uniformized product, followed by stirring and mixing. Furthermore, the mixture was cooled to 25°C while being stirred, to obtain a skin cleansing composition. Then, according to the following method, the viscosity and pH of each of the obtained compositions were measured.

### (Compositions 2 to 9)

According to the formulations of Tables 1 and 2, skin cleansing compositions (compositions 2 to 9) were manufactured as with the composition 1. Then, according to the following method, the viscosity and pH of each of the obtained compositions were measured.

The results are shown in Tables 1 and 2.

### <<Viscosity>>

A B type viscometer (TVB-10 type viscometer, manufactured by Toki Sangyo Co., Ltd.) was used to measure the viscosity under conditions of 25°C, rotor No. 1, (number of rotations) of 60 rpm, and a measuring time of 1 minute.

### <<pH>>

A pH electrode (manufactured by Horiba, Ltd., model number F-22) was used to measure the pH at 25°C.

**[Table 1]**

| | | Composition 1 | Composition 2 | Composition 3 | Composition 4 | Composition 5 | Composition 6 | Composition 7 |
|---|---|---|---|---|---|---|---|---|
| (A) | Sodium cocoyl glutamate ^{*2} | | | | | 2.56 | 8.00 | |
| | Polyoxyethylene (4.5) lauryl ether acetate ^{*1} | 8.70 | 1.09 | 16.31 | 2.18 | | | |
| | Lauric acid | | | | 4.88 | | 4.88 | 6.50 |
| | Myristic acid | | | | 0.83 | | 0.83 | 1.10 |
| | Palmitic acid | | | | 0.30 | | 0.30 | 0.40 |
| | Potassium hydroxide (48% aqueous solution) | 1.87 | 0.25 | 3.75 | 3.87 | 0.06 | 3.42 | 4.53 |
| (B) | Propylene glycol | 5.00 | 10.00 | 5.00 | 5.00 | 5.00 | 5.00 | 5.00 |
| | Glycerin | 5.00 | 20.00 | 5.00 | 5.00 | 5.00 | 5.00 | 5.00 |
| | Sorbitol | 5.00 | 5.00 | 5.00 | 5.00 | 5.00 | 5.00 | 5.00 |
| | Phenoxyethanol | 0.20 | 0.20 | 0.20 | 0.20 | 0.20 | 0.20 | 0.20 |
| | Disodium ethylenediaminetetraacetate | 0.10 | 0.10 | 0.10 | 0.10 | 0.10 | 0.10 | 0.10 |
| | Purified water | Balance | Balance | Balance | Balance | Balance | Balance | Balance |
| | Total | 100.00 | 100.00 | 100.00 | 100.00 | 100.00 | 100.00 | 100.00 |
| (A) | Amount of component (A) in terms of acid | 8.70 | 1.09 | 16.31 | 2.18 | 2.40 | 7.50 | 0.00 |
| (C) | Amount of moisture | 75.10 | 63.49 | 66.59 | 74.67 | 82.11 | 69.06 | 74.53 |
| Viscosity (mPa·s) | | 5.8 | 5.2 | 9.3 | 5.7 | 5.4 | 5.7 | 5.6 |
| pH(25°C) | | 10.0 | 10.0 | 10.0 | 10.0 | 6.9 | 9.1 | 10.0 |

| | | | | | | | | |
|---|---|---|---|---|---|---|---|---|
| *1: AKYPO RLM-45CA, manufactured by Kao Corporation *2: AMISOFT CS-22B, manufactured by Ajinomoto Co., Inc., effective part: 8% by mass (amounts in Table are effective amounts) | | | | | | | | |

**[Table 2]**

| | | Composition 8 | Composition 9 |
|---|---|---|---|
| (A) | Cocoyl glycine | | 11.20 |
| | Polyoxyethylene (4.5) lauryl ether acetate ^{*1} | 2.30 | |
| | Laureth 4-carboxylic acid ^{*3} | 0.10 | |
| | Lauric acid | 4.50 | |
| | Myristic acid | 0.75 | |
| | Palmitic acid | 0.30 | 0.30 |
| | Stearic acid | | 0.30 |
| | Lauryl hydroxy sulfobetaine ^{*4} | 1.20 | |
| | Lauroamphoacetate | | 1.80 |
| | Polyoxyethylene polyoxypropylene decyl tetradecyl ether | | 0.02 |
| | Potassium hydroxide (48% aqueous solution) | 3.20 | 1.60 |
| | Sodium hydroxide (48% aqueous solution) | | 0.15 |
| | Arginine | 1.00 | |
| (B) | Glycerin | | 6.00 |
| | Sorbitol | 4.00 | |
| | Propylene glycol | 2.00 | 6.00 |
| | Dipropylene glycol | 3.00 | |
| | Polyethylene glycol ^{*5} | 4.00 | |
| | Citric acid | | 0.10 |
| | Phenoxyethanol | 0.20 | |
| | Methylisothiazolinone | | 0.005 |
| | Disodium ethylenediaminetetraacetate | 0.10 | 0.05 |
| | Dibutylhydroxytoluene | | 0.05 |
| | Perfume | 0.10 | 0.10 |
| | Purified water | Balance | Balance |
| | Total | 100.00 | 100.00 |
| (A) | Amount of component (A) in terms of acid | 2.40 | 11.20 |
| (C) | Amount of moisture | 74.914 | 73.235 |
| Viscosity (mPa·s) | | 8.6 | 7.4 |
| pH(25°C) | | 9.2 | 7.7 |

| | | | |
|---|---|---|---|
| *1: AKYPO RLM-45CA, manufactured by Kao Corporation *3: AKYPO LM-26C, manufactured by Kao Corporation *4: Amphitol 20HD, manufactured by Kao Corporation, effective part: 30% (amounts in Table are effective amounts) *5: PEG6000 (manufactured by NOF Corporation, molecular weight: 8,800) | | | |

### [Examples 1-1 to 9-2, Comparative Example 1-1 to Comparative Example 9-1, Reference Example 1-1 to Reference Example 9-1]

Pump type non-aerosol-type containers (containers described in JP-A-2009-202122) in which volume ratios (air/composition) in gas-liquid mixing parts were different from each other, i.e., containers 1 to 8 were used as non-aerosol-type containers, and an environmental temperature was set to 25°C. Each of the containers was filled with the composition obtained in Production Example 1 according to the descriptions of Tables 3 to 7, to obtain an article for skin cleansing agent.

Each of the containers included two meshes (#200/opening size: 77 µm, #305/opening size: 44 µm) in a foam micronizing part. A pump head was pressed so that 1 mL of the composition was delivered into a gas-liquid mixing chamber at a discharge speed of 30 mm/s by a liquid cylinder by one push; air of a predetermined volume ratio was delivered into the gas-liquid mixing chamber at a discharge speed of 30 mm/s by an air cylinder by one push; the composition and the air were mixed to form foams; and the foams were micronized through the foam micronizing part, followed by discharging the foams from a discharge outlet.

According to the following method, the obtained article for skin cleansing agent was evaluated and measured.

The results are shown in Tables 3 to 7.

### <<Initial Foam Elastic Force>>

Each of the obtained articles for skin cleansing agent was evaluated by three specialized panelists. Specifically, first, foams were discharged onto a wet palm of one hand from the discharge outlet of the container (liquid volume: 2 mL). Then, the other hand was immediately pressed onto the discharged foams from above, and the degree of the elasticity of the foams felt by the other hand was evaluated according to the following criteria.

Based on the evaluations provided by the three specialized panelists, evaluations determined by the deliberations are shown in Tables 3 to 5 (in Table, "-" means no measurement).
5: very elastic
4: elastic
3: slightly elastic
2: slightly inelastic
1: inelastic

### <<Density of Foams>>

Each of the obtained articles for skin cleansing agent was evaluated by three specialized panelists. Specifically, first, foams were discharged onto a wet palm of one hand from the discharge outlet of the container (liquid volume: 2 mL). Then, the discharged foams were immediately held between the one hand and the other hand. The hands were rubbed together twenty times, and the degree of the density (fineness) of the foams felt by the hands was evaluated according to the following criteria.

Based on the evaluations provided by the three specialized panelists, evaluations determined by the deliberations are shown in Tables 3 to 5.
5: very dense
**4:** dense
3: slightly dense
**2:** slightly less dense
1: less dense

### <<Measurements of Number of Foams and Total Length of Foam Interface>>

A foam diameter analyzer (DFA 100, Foam Analysis, manufactured by KRUSS) was used as a measuring device, and foams discharged from the discharge outlet of the container was placed on a plate. Foams present in an observation region (1,280 × 1,240 pixels) were observed according to image processing.

The number of foams present in the observation region was confirmed, and the total length of the confirmed foam interface was measured. The number of foams and the total length of the foam interface are shown in Table 6 as the average value of the results of three measurements.

The number of foams and the total length of the foam interface when the container 1 (the volume ratio (air/composition) in the gas-liquid mixing part = 13/1) was used were taken as a standard (100%), and the increasing rates (%) of the number of foams and the total length of the foam interface when each of the containers was used were determined.

### <<Measurement of Viscosity Coefficient>>

MCR 502 (Modular Compact Rheometer, manufactured by Anton Paar, Measurement Cell: P-PTD200, Measurement System: PP50) was used as a device for measuring a viscosity coefficient, and measurement conditions of Gap: 0.5 mm and temperature: 30°C were set.

First, foams discharged from the discharge outlet of the container was placed on a plate, and a viscosity coefficient (Pa·s) at an elapse of 5 seconds after shear was applied and a shear rate of 1,000 (1/s) was reached, as shown in Figure 1 was measured.

The viscosity coefficient when the container 1 (the volume ratio (air/composition) in the gas-liquid mixing part = 13/1) was used was taken as a standard (100%), and the increasing rate (%) of the viscosity coefficient when each of the containers was used was determined.

### <<Measurement of Air Ratio>>

The foams were discharged from the discharge outlet of the container (liquid volume: 2 mL). The volume of each of the foam-like skin cleansing compositions at an elapse of 5 seconds after the foams were discharged was measured. The ratio of the volume to the liquid volume of 2 mL was determined, and the average value of ten discharges was calculated. The obtained value is a value of the volume ratio of the volume of the foam-like skin cleansing composition (Y) to the volume of the liquid skin cleansing composition (Y) (foam-like volume/liquid volume), and means the air ratio of each of the skin cleansing compositions. It was confirmed that the values were separated by ±0.5 from the value of the volume ratio (air/composition) in the gas-liquid mixing part, and approximate to each other.

**[Table 3]**

| | Comparativ e Example 1-1 | Reference Example 1-1 | Example 1-1 | Example 1-2 | Example 1-3 | Reference Example 1-2 | Comparat ive Example 2-1 | Referenc e Example 2-1 | Comparat ive Example 3-1 | Referenc e Example 3-1 | Comparativ e Example 4-1 | Reference Exampe 4-1 | Example 4-1 | Example 4-2 | Example 4-3 | Reference Example 4-2 | Reference Example 4-3 |
|---|---|---|---|---|---|---|---|---|---|---|---|---|---|---|---|---|---|
| Non-aerosol-type container (air/compositi on) | Container 1 (13/1) | Container 2 (17/1) | Containe r 3 (20/1) | Containe r 4 (24/1) | Containe r 5 (28/1) | Container 6 (32/1) | Container 1 (13/1) | Container 2 (17/1) | Container 1 (13/1) | Container 2 (17/1) | Container 1 (13/1) | Container 2 (17/1) | Container 3 (20/1) | Container 4 (24/1) | Container 5 (28/1) | Container 6 (32/1) | Container 7 (36/1) |
| Skin cleansing composition | Compositio n 1 | Compositi on 1 | Composi tion 1 | Composi tion 1 | Composi tion 1 | Compositio n 1 | Compositi on 2 | Compositi on 2 | Compositi on 3 | Compositi on 3 | Compositio n 4 | Compositio n 4 | Compositi on 4 | Compositi on 4 | Compositi on 4 | Compositio n 4 | Compositi on 4 |
| Initial foam elastic force | 2 | 2 | 3 | 4 | 3 | 3 | 1 | 2 | 2 | 3 | 1 | 2 | 2 | 2 | 3 | 3 | 3 |
| Density foams | 2 | 4 | 4 | 5 | 3 | 4 | 1 | 2 | 3 | 4 | 2 | 2 | 2 | 2 | 3 | 3 | 3 |

**[Table 4]**

| | Comparati ve Example 5-1 | Reference Example 5-1 | Example 5-1 | Example 5-2 | Example 5-3 | Reference Example 5-2 | Reference Example 5-3 | Comparati ve Example 6-1 | Reference Example 6-1 | Example 6-1 | Example 6-2 | Example 6-3 | Reference Example 6-2 | Reference Example 6-3 |
|---|---|---|---|---|---|---|---|---|---|---|---|---|---|---|
| Non-aerosol-type container (air/composition) | Container 1 (13/1) | Container 2 (17/1) | Container 3 (20/1) | Container 4 (24/1) | Container 5 (28/1) | Container 6 (32/1) | Container 7 (36/1) | Container 1 (13/1) | Container 2 (17/1) | Container 3 (20/1) | Container 4 (24/1) | Container 5 (28/1) | Container 6 (32/1) | Container 7 (36/1) |
| Skin cleansing composition | Compositio n 5 | Compositi on 5 | Compositi on 5 | Compositi on 5 | Compositi on 5 | Compositio n 5 | Compositio n 5 | Compositio n 6 | Compositi on 6 | Compositi on 6 | Compositi on 6 | Compositi on 6 | Compositio n 6 | Compositio n 6 |
| Initial foam elastic force | 1 | 2 | 3 | 4 | 4 | 4 | 4 | 1 | 2 | 3 | 3 | 4 | 4 | 4 |
| Density of foams | 2 | 2 | 2 | 3 | 4 | 4 | 4 | 2 | 2 | 2 | 2 | 3 | 3 | 2 |

**[Table 5]**

| | Comparati ve Example 7-1 | Comparati ve Example 7-2 | Comparati ve Example 7-3 | Comparati ve Example 7-4 | Comparati ve Example 7-5 | Comparati ve Example 7-6 | Comparati ve Example 7-7 | Comparati ve Example 8-1 | Referenc e Example 8-1 | Example 8-1 | Example 8-2 | Comparati ve Example 9-1 | Referenc e Example 9-1 | Example 9-1 | Example 9-2 |
|---|---|---|---|---|---|---|---|---|---|---|---|---|---|---|---|
| Non-aerosol-type container (air/composition) | Container 1 (13/1) | Container 2 (17/1) | Container 3 (20/1) | Container 4 (24/1) | Container 5 (28/1) | Container 6 (32/1) | Container 7 (36/1) | Container 1 (13/1) | Container 2 (17/1) | Container 3 (20/1) | Container 4 (24/1) | Container 1 (13/1) | Container 2 (17/1) | Container 3 (20/1) | Container 4 (24/1) |
| Skin cleansing composition | Compositi on 7 | Compositi on 7 | Compositi on 7 | Compositi on 7 | Compositi on 7 | Compositi on 7 | Compositi on 7 | Compositi on 8 | Compositi on 8 | Compositi on 8 | Compositi on 8 | Compositi on 9 | Compositi on 9 | Compositi on 9 | Compositi on 9 |
| Initial foam elastic force | 1 | 1 | 2 | 2 | 3 | 3 | 3 | 3 | 4 | 5 | 5 | 3 | 4 | 4 | 5 |
| Density of foams | 1 | 1 | 1 | 2 | 2 | 2 | 2 | 3 | 4 | 5 | 5 | 2 | 4 | 4 | 5 |

**[Table 6]**

| | Comparati ve Example 7-1 | Comparati ve Example 7-2 | Comparati ve Example 7-3 | Comparati ve Example 1-1 | Referenc e Example 1-1 | Example 1-1 | Comparati ve Example 4-1 | Referenc e Example 4-1 | Example 4-1 | Comparati ve Example 5-1 | Referenc e Example 5-1 | Example 5-1 | Comparati ve Example 6-1 | Referenc e Example 6-1 | Example 6-1 |
|---|---|---|---|---|---|---|---|---|---|---|---|---|---|---|---|
| Non-aerosol-type container (air/composition) | Container 1 (13/1) | Container 2 (17/1) | Container 3 (20/1) | Container 1 (13/1) | Container 2 (17/1) | Container 3 (20/1) | Container 1 (13/1) | Container 2 (17/1) | Container 3 (20/1) | Container 1 (13/1) | Container 2 (17/1) | Container 3 (20/1) | Container 1 (13/1) | Container 2 (17/1) | Container 3 (20/1) |
| Skin cleansing composition | Compositi on 7 | Compositi on 7 | Compositi on 7 | Compositi on 1 | Compositi on 1 | Compositi on 1 | Compositi on 4 | Compositi on 4 | Compositi on 4 | Compositi on 5 | Compositi on 5 | Compositi on 5 | Compositi on 6 | Compositi on 6 | Compositi on 6 |
| Number of foams (foam) | 1415 | 1413 | 1457 | 2223 | 2524 | 2773 | 1272 | 1490 | 2122 | 1335 | 1517 | 1932 | 1487 | 1664 | 2405 |
| Increasing rate of number of foams (%) | - | - | - | - | 13 | 25 | - | 17 | 67 | - | 13 | 45 | - | 12 | 62 |
| Total length of foam interface (× 10⁵ µm) | 5.51 | 5.57 | 5.62 | 7.62 | 8.06 | 9.11 | 5.20 | 6.28 | 7.98 | 5.59 | 6.24 | 7.03 | 6.37 | 6.9 | 8.72 |
| Increasing rate of total length of foam interface (%) | - | - | - | - | 6 | 20 | - | 21 | 53 | - | 12 | 26 | - | 8 | 37 |

**[Table 7]**

| | Comparati ve Example 1-1 | Reference Example 1-1 | Example 1-1 | Example 1-2 | Example 1-3 | Reference Example 1-2 | Comparati ve Example 8-1 | Reference Example 8-1 | Example 8-1 | Example 8-2 | Example 8-3 | Reference Example 8-2 |
|---|---|---|---|---|---|---|---|---|---|---|---|---|
| Non-aerosol-type container (air/composition) | Container 1 (13/1) | Container 2 (17/1) | Container 3 (20/1) | Container 4 (24/1) | Container 5 (28/1) | Container 6 (32/1) | Container 1 (13/1) | Container 2 (17/1) | Container 3 (20/1) | Container 4 (24/1) | Container 5 (28/1) | Container 6 (32/1) |
| Skin cleansing composition | Compositi on 1 | Compositi on 1 | Compositi on 1 | Compositi on 1 | Compositi on 1 | Compositi on 1 | Compositi on 8 | Compositi on 8 | Compositi on 8 | Compositi on 8 | Compositi on 8 | Compositi on 8 |
| Viscosity coefficient | 6.9E-02 | 8.9E-02 | 1.2E-01 | 1.4E-01 | 1.5E-01 | 9.7E-02 | 1.2E-01 | 1.7E-01 | 2.3E-01 | 2.5E-01 | 2.5E-01 | 2.8E-01 |
| Increasing rate of viscosity coefficient (based on container 1) | - | 28 | 78 | 106 | 117 | 39 | - | 37 | 84 | 103 | 102 | 126 |

### Reference Signs List

P: liquid skin cleansing composition
Q: air
r: interfaces of foams
M: measurement point at an elapse of 5 seconds after a shear rate of 1,000 (1/s) is reached when a viscosity coefficient is measured.

## Claims

1. An article for skin cleansing agent comprising a non-aerosol-type container filled with a skin cleansing composition,
wherein:
the non-aerosol-type container comprises a gas-liquid mixing part, a foam micronizing part, and a discharge outlet, and has a mechanism for discharging air and the skin cleansing composition at a volume ratio (air/composition) of 20/1 or more and 30/1 or less from the discharge outlet; and
the skin cleansing composition comprises the following components (A) to (C):
(A) 0.5% by mass or more and 18% by mass or less, in terms of acid, of one or more anionic surfactants selected from the group consisting of an N-acylamino acid salt, and a polyoxyethylene alkyl ether carboxylic acid or a salt thereof;
(B) 2% by mass or more and 50% by mass or less of one or more polyols selected from the group consisting of glycerin, sorbitol, propylene glycol, butylene glycol, dipropylene glycol, and polyethylene glycol having a mass average molecular weight of 20,000 or less; and
(C) 40% by mass or more and 90% by mass or less of water;
wherein a total content of the components (A) and (B) in the skin cleansing composition is 10% by mass or more and 60% by mass or less;
wherein the mass average molecular weight of the polyethylene glycol is measured by gel permeation chromatography.

2. The article for skin cleansing agent according to claim 1, wherein a content of a fatty acid salt in the skin cleansing composition is 0.5% by mass or more and 10% by mass or less in terms of acid.

3. The article for skin cleansing agent according to claims 1 or 2, wherein a content of an amphoteric surfactant (D) in the skin cleansing composition is 0.1% by mass or more and 5% by mass or less.

4. The article for skin cleansing agent according to any one of claims 1 to 3, wherein a content of a non-ionic surfactant (E) in the skin cleansing composition is 0.1% by mass or more and 5% by mass or less.

5. The article for skin cleansing agent according to any one of claims 1 to 4, wherein the content of a thickener in the skin cleansing composition is 5% by mass or less.

6. The article for skin cleansing agent according to any one of claims 1 to 5, wherein a viscosity at 25°C of the skin cleansing composition is 2 mPa·s or more and 20 mPa·s or less.

7. The article for skin cleansing agent according to any one of claims 1 to 6, wherein the viscosity coefficient of the skin cleansing composition is a value at an elapse of 5 seconds after a shearing force is applied to the discharged skin cleansing composition and a shear rate of 1,000 (1/s) is reached, and is 6 × 10⁻² Pa·s or more.

8. The article for skin cleansing agent according to any one of claims 1 to 7, wherein a pH at 25°C of the skin cleansing composition is 5 or more and 11 or less.

9. The article for skin cleansing agent according to any one of claims 1 to 8, wherein a content of ethanol in the skin cleansing composition is 5% by mass or less.

10. The article for skin cleansing agent according to any one of claims 1 to 9, wherein the non-aerosol-type container is a pump type container.

11. The article for skin cleansing agent according to any one of claims 1 to 10, wherein the foam micronizing part in the non-aerosol container has a porous member having a mesh with the opening of 16 to 180 µm.

12. The article for skin cleansing agent according to any one of claims 1 to 11,
wherein:
the non-aerosol-type container has a mechanism for discharging air and the skin cleansing composition at a volume ratio (air/composition) of 20/1 or more and 24/1 or less from the discharge outlet;
the skin cleansing composition comprises the following components (A) to (E):
(A) 2.0% by mass or more and 12% by mass or less, in terms of acid, of one or more anionic surfactants selected from the group consisting of an N-acylamino acid salt, and a polyoxyethylene alkyl ether carboxylic acid or a salt thereof;
(B) 9% by mass or more and 20% by mass or less of one or more polyols selected from the group consisting of glycerin, sorbitol, propylene glycol, butylene glycol, dipropylene glycol, and polyethylene glycol having a mass average molecular weight of 20,000 or less;
(C) 45% by mass or more and 85% by mass or less of water;
(D) 0.8% by mass or more and less than 2% by mass of an amphoteric surfactant; and
(E) 0.001% by mass or more and 0.5% by mass or less of a non-ionic surfactant; and wherein
a mass ratio of the total content of the anionic surfactants containing the component (A) to the content of the component (D) (anionic surfactant/(D)) is 5.5 or more and 20 or less.

13. Use of the article for skin cleansing agent according to any one of claims 1 to 12 as a skin cleansing agent.

14. A method for cleansing skin, comprising
filling a skin cleansing composition in a pump type of a non-aerosol-type container,
subsequently discharging a foam-like skin cleansing composition from the discharge outlet and
applying the discharged foam-like skin cleansing composition to the skin directly;
wherein
the non-aerosol-type container comprises a gas-liquid mixing part, a foam micronizing part, and a discharge outlet, and has a mechanism for discharging air and the skin cleansing composition at a volume ratio (air/composition) of 20/1 or more and 30/1 or less from the discharge outlet;
the skin cleansing composition comprises the following components (A) to (C):
(A) 0.5% by mass or more and 18% by mass or less, in terms of acid, of one or more anionic surfactants selected from the group consisting of an N-acylamino acid salt, and a polyoxyethylene alkyl ether carboxylic acid or a salt thereof;
(B) 2% by mass or more and 50% by mass or less of one or more polyols selected from the group consisting of glycerin, sorbitol, propylene glycol, butylene glycol, dipropylene glycol, and polyethylene glycol having a mass average molecular weight of 20,000 or less; and
(C) 40% by mass or more and 90% by mass or less of water;
wherein a total content of the components (A) and (B) in the skin cleansing composition is 10% by mass or more and 60% by mass or less;
wherein the mass average molecular weight of the polyethylene glycol is measured by gel permeation chromatography.

## Patentansprüche

1. Artikel für ein Hautreinigungsmittel, umfassend einen nicht-aerosolartigen Behälter, der mit einer Hautreinigungszusammensetzung gefüllt ist,
wobei:
der nicht-aerosolartige Behälter einen Gas-Flüssigkeits-Mischteil, einen Schaum-Mikronisierungsteil und einen Auslass umfasst und einen Mechanismus zum Abgeben von Luft und der Hautreinigungszusammensetzung in einem Volumenverhältnis (Luft/Zusammensetzung) von 20/1 oder mehr und 30/1 oder weniger aus dem Auslass aufweist; und
die Hautreinigungszusammensetzung die folgenden Komponenten (A) bis (C) umfasst:
(A) 0,5 Massen-% oder mehr und 18 Massen-% oder weniger, bezogen auf die Säure, eines oder mehrerer anionischer Tenside, ausgewählt aus der Gruppe bestehend aus einem N-Acylaminosäuresalz, und einer Polyoxyethylenalkylethercarbonsäure oder einem Salz davon;
(B) 2 Massen-% oder mehr und 50 Massen-% oder weniger eines oder mehrerer Polyole, ausgewählt aus der Gruppe bestehend aus Glycerin, Sorbit, Propylenglykol, Butylenglykol, Dipropylenglykol und Polyethylenglykol mit einem massenmittleren Molekulargewicht von 20.000 oder weniger; und
(C) 40 Massen-% oder mehr und 90 Massen-% oder weniger Wasser;
wobei der Gesamtgehalt der Komponenten (A) und (B) in der Hautreinigungszusammensetzung 10 Massen-% oder mehr und 60 Massen-% oder weniger beträgt;
wobei das massenmittlere Molekulargewicht des Polyethylenglykols durch Gelpermeationschromatographie gemessen wird.

2. Artikel für ein Hautreinigungsmittel gemäß Anspruch 1, wobei ein Gehalt an Fettsäuresalz in der Hautreinigungszusammensetzung 0,5 Massen-% oder mehr und 10 Massen-% oder weniger, bezogen auf die Säure, beträgt.

3. Artikel für ein Hautreinigungsmittel gemäß Anspruch 1 oder 2, wobei ein Gehalt an einem amphoteren Tensid (D) in der Hautreinigungszusammensetzung 0,1 Massen-% oder mehr und 5 Massen-% oder weniger beträgt.

4. Artikel für ein Hautreinigungsmittel gemäß mindestens einem der Ansprüche 1 bis 3, wobei ein Gehalt an einem nichtionischen Tensid (E) in der Hautreinigungszusammensetzung 0,1 Massen-% oder mehr und 5 Massen-% oder weniger beträgt.

5. Artikel für ein Hautreinigungsmittel gemäß mindestens einem der Ansprüche 1 bis 4, wobei der Gehalt an einem Verdickungsmittel in der Hautreinigungszusammensetzung 5 Massen-% oder weniger beträgt.

6. Artikel für ein Hautreinigungsmittel gemäß mindestens einem der Ansprüche 1 bis 5, wobei eine Viskosität der Hautreinigungszusammensetzung bei 25°C 2 mPa·s oder mehr und 20 mPa·s oder weniger beträgt.

7. Artikel für ein Hautreinigungsmittel gemäß mindestens einem der Ansprüche 1 bis 6, wobei der Viskositätskoeffizient der Hautreinigungszusammensetzung ein Wert nach Ablauf von 5 Sekunden nach Aufbringen einer Scherkraft auf die abgegebene Hautreinigungszusammensetzung und Erreichen einer Schergeschwindigkeit von 1.000 (1/s) ist, und 6 × 10⁻² Pa·s oder mehr beträgt.

8. Artikel für ein Hautreinigungsmittel gemäß mindestens einem der Ansprüche 1 bis 7, wobei ein pH-Wert der Hautreinigungszusammensetzung bei 25°C 5 oder mehr und 11 oder weniger beträgt.

9. Artikel für ein Hautreinigungsmittel gemäß mindestens einem der Ansprüche 1 bis 8, wobei der Ethanolgehalt in der Hautreinigungszusammensetzung 5 Massen-% oder weniger beträgt.

10. Artikel für ein Hautreinigungsmittel gemäß mindestens einem der Ansprüche 1 bis 9, wobei der nicht-aerosolartige Behälter ein Pumpbehälter ist.

11. Artikel für ein Hautreinigungsmittel gemäß mindestens einem der Ansprüche 1 bis 10, wobei der Schaum-Mikronisierungsteil in dem nicht-aerosolartigen Behälter ein poröses Element mit einer Maschenweite von 16 bis 180 µm aufweist.

12. Artikel für ein Hautreinigungsmittel gemäß mindestens einem der Ansprüche 1 bis 11,
wobei:
der nicht-aerosolartige Behälter einen Mechanismus zum Abgeben von Luft und der Hautreinigungszusammensetzung in einem Volumenverhältnis (Luft/Zusammensetzung) von 20/1 oder mehr und 24/1 oder weniger aus dem Auslass aufweist;
die Hautreinigungszusammensetzung die folgenden Komponenten (A) bis (E) umfasst:
(A) 2,0 Massen-% oder mehr und 12 Massen-% oder weniger, bezogen auf die Säure, eines oder mehrerer anionischer Tenside, ausgewählt aus der Gruppe bestehend aus einem N-Acylaminosäuresalz und einer Polyoxyethylenalkylethercarbonsäure oder einem Salz davon;
(B) 9 Massen-% oder mehr und 20 Massen-% oder weniger eines oder mehrerer Polyole, ausgewählt aus der Gruppe bestehend aus Glycerin, Sorbit, Propylenglykol, Butylenglykol, Dipropylenglykol und Polyethylenglykol mit einem massenmittleren Molekulargewicht von 20.000 oder weniger;
(C) 45 Massen-% oder mehr und 85 Massen-% oder weniger Wasser;
(D) 0,8 Massen-% oder mehr und weniger als 2 Massen-% eines amphoteren Tensids; und
(E) 0,001 Massen-% oder mehr und 0,5 Massen-% oder weniger eines nichtionischen Tensids; und wobei
das Massenverhältnis des Gesamtgehalts der anionischen Tenside, die die Komponente (A) enthalten, zum Gehalt der Komponente (D) (anionisches Tensid/(D)) 5,5 oder mehr und 20 oder weniger beträgt.

13. Verwendung des Artikels für ein Hautreinigungsmittel gemäß mindestens einem der Ansprüche 1 bis 12 als ein Hautreinigungsmittel.

14. Verfahren zur Reinigung von Haut, umfassend
Einfüllen einer Hautreinigungszusammensetzung in einen Pumpbehälter eines nicht-aerosolartigen Behälters,
anschließendes Abgeben einer schaumartigen Hautreinigungszusammensetzung aus dem Auslass und
direktes Aufbringen der abgegebenen schaumartigen Hautreinigungszusammensetzung auf die Haut;
wobei
der nicht-aerosolartige Behälter einen Gas-Flüssigkeits-Mischteil, einen Schaum-Mikronisierungsteil und einen Auslass umfasst und einen Mechanismus zum Abgeben von Luft und der Hautreinigungszusammensetzung in einem Volumenverhältnis (Luft/Zusammensetzung) von 20/1 oder mehr und 30/1 oder weniger aus dem Auslass aufweist; und
die Hautreinigungszusammensetzung die folgenden Komponenten (A) bis (C) umfasst:
(A) 0,5 Massen-% oder mehr und 18 Massen-% oder weniger, bezogen auf die Säure, eines oder mehrerer anionischer Tenside, ausgewählt aus der Gruppe bestehend aus einem N-Acylaminosäuresalz, und einer Polyoxyethylenalkylethercarbonsäure oder einem Salz davon;
(B) 2 Massen-% oder mehr und 50 Massen-% oder weniger eines oder mehrerer Polyole, ausgewählt aus der Gruppe bestehend aus Glycerin, Sorbit, Propylenglykol, Butylenglykol, Dipropylenglykol und Polyethylenglykol mit einem massenmittleren Molekulargewicht von 20.000 oder weniger; und
(C) 40 Massen-% oder mehr und 90 Massen-% oder weniger Wasser;
wobei der Gesamtgehalt der Komponenten (A) und (B) in der Hautreinigungszusammensetzung 10 Massen-% oder mehr und 60 Massen-% oder weniger beträgt;
wobei das massenmittlere Molekulargewicht des Polyethylenglykols durch Gelpermeationschromatographie gemessen wird.

## Revendications

1. Article destiné à un agent de nettoyage de la peau comprenant un récipient de type non-aérosol rempli d'une composition de nettoyage de la peau,
dans lequel :
le récipient de type non-aérosol comprend une partie de mélange de gaz-liquide, une partie de micronisation de mousse et une sortie d'évacuation, et présente un mécanisme destiné à évacuer l'air et la composition de nettoyage de la peau à un rapport volumique (air/composition) de 20/1 ou plus et de 30/1 ou moins par la sortie d'évacuation ; et
la composition de nettoyage de la peau comprend les composants (A) à (C) suivants :
(A) 0,5 % en masse ou plus et 18 % en masse ou moins, en termes d'acide, d'un ou plusieurs tensioactifs anioniques sélectionnés dans le groupe consistant en un sel d'acide N-acylamino et un acide carboxylique d'éther alkylique de polyoxyéthylène ou un sel de celui-ci ;
(B) 2 % en masse ou plus et 50 % en masse ou moins d'un ou plusieurs polyols sélectionnés dans le groupe consistant en glycérine, sorbitol, propylène glycol, butylène glycol, dipropylène glycol et polyéthylène glycol présentant un poids moléculaire moyen en masse de 20 000 ou moins ; et
(C) 40 % en masse ou plus et 90 % en masse ou moins d'eau ; dans lequel une teneur totale des composants (A) et (B) dans la composition de nettoyage de la peau est de 10 % en masse ou plus et 60 % en masse ou moins ;
dans lequel le poids moléculaire moyen en masse du polyéthylène glycol est mesuré par chromatographie par perméation sur gel.

2. Article destiné à un agent de nettoyage de la peau selon la revendication 1, dans lequel une teneur en sel d'acides gras dans la composition de nettoyage de la peau est de 0,5 % en masse ou plus et 10 % en masse ou moins en termes d'acide.

3. Article destiné à un agent de nettoyage de la peau selon la revendication 1 ou la revendication 2, dans lequel une teneur d'un tensioactif amphotère (D) dans la composition de nettoyage de la peau est de 0,1 % en masse ou plus et 5 % en masse ou moins.

4. Article destiné à un agent de nettoyage de la peau selon l'une quelconque des revendications 1 à 3, dans lequel une teneur en un tensioactif non ionique (E) dans la composition de nettoyage de la peau est de 0,1 % en masse ou plus et 5 % en masse ou moins.

5. Article destiné à un agent de nettoyage de la peau selon l'une quelconque des revendications 1 à 4, dans lequel la teneur en épaississant dans la composition de nettoyage de la peau est de 5 % en masse ou moins.

6. Article destiné à un agent de nettoyage de la peau selon l'une quelconque des revendications 1 à 5, dans lequel une viscosité à 25 °C de la composition de nettoyage de la peau est de 2 mPa·s ou plus et 20 mPa·s ou moins.

7. Article destiné à un agent de nettoyage de la peau selon l'une quelconque des revendications 1 à 6, dans lequel le coefficient de viscosité de la composition de nettoyage de la peau est une valeur à un écoulement de 5 secondes après qu'une force de cisaillement a été appliquée à la composition de nettoyage de la peau évacuée et qu'un taux de cisaillement de 1 000 (1/s) a été atteint, et est de 6 x 10⁻² Pa·s ou plus.

8. Article destiné à un agent de nettoyage de la peau selon l'une quelconque des revendications 1 à 7, dans lequel un pH à 25 °C de la composition de nettoyage de la peau est de 5 ou plus et 11 ou moins.

9. Article destiné à un agent de nettoyage de la peau selon l'une quelconque des revendications 1 à 8, dans lequel une teneur en éthanol dans la composition de nettoyage de la peau est de 5 % en masse ou moins.

10. Article destiné à un agent de nettoyage de la peau selon l'une quelconque des revendications 1 à 9, dans lequel le récipient de type non-aérosol est un récipient de type pompe.

11. Article destiné à un agent de nettoyage de la peau selon l'une quelconque des revendications 1 à 10, dans lequel la partie de micronisation de mousse dans le récipient non-aérosol présente un élément poreux présentant un tamis avec l'ouverture de 16 à 180 µm.

12. Article destiné à un agent de nettoyage de la peau selon l'une quelconque des revendications 1 à 11,
dans lequel :
le récipient de type non-aérosol présente un mécanisme destiné à évacuer de l'air et la composition de nettoyage de la peau à un rapport volumique (air/composition) de 20/1 ou plus et de 24/1 ou moins par la sortie d'évacuation ;
la composition de nettoyage de la peau comprend les composants (A) à (E) suivants.
(A) 2,0 % en masse ou plus et 12 % en masse ou moins, en termes d'acide, d'un ou plusieurs tensioactifs anioniques sélectionnés dans le groupe consistant en un sel d'acide N-acylamino et un acide carboxylique d'éther alkylique de polyoxyéthylène ou un sel de celui-ci ;
(B) 9 % en masse ou plus et 20 % en masse ou moins d'un ou plusieurs polyols sélectionnés dans le groupe consistant en glycérine, sorbitol, propylène glycol, butylène glycol, dipropylène glycol et polyéthylène glycol présentant un poids moléculaire moyen en masse de 20 000 ou moins ;
(C) 45 % en masse ou plus et 85 % en masse ou moins d'eau ;
(D) 0,8 % en masse ou plus et moins de 2 % en masse d'un tensioactif amphotère ; et
(E) 0,001 % en masse ou plus et 0,5 % en masse ou moins d'un tensioactif non ionique ; et dans lequel un rapport massique de la teneur totale des tensioactifs anioniques contenant le composant (A) sur la teneur du composant (D) (tensioactif anionique/(D)) est de 5,5 ou plus et 20 ou moins.

13. Utilisation de l'article destiné à un agent de nettoyage de la peau selon l'une quelconque des revendications 1 à 12 en tant qu'agent de nettoyage de la peau.

14. Procédé pour nettoyage de la peau, comprenant
le remplissage d'une composition de nettoyage de la peau dans un type pompe d'un récipient de type non-aérosol,
l'évacuation ultérieure d'une composition de nettoyage de la peau de type mousse par la sortie d'évacuation et
l'application, directement sur la peau, de la composition de nettoyage de la peau de type mousse évacuée ;
dans lequel
le récipient de type non-aérosol comprend une partie de mélange de gaz-liquide, une partie de micronisation de mousse et une sortie d'évacuation, et présente un mécanisme destiné à évacuer l'air et la composition de nettoyage de la peau à un rapport volumique (air/composition) de 20/1 ou plus et de 30/1 ou moins par la sortie d'évacuation ;
la composition de nettoyage de la peau comprend les composants (A) à (C) suivants.
(A) 0,5 % en masse ou plus et 18 % en masse ou moins, en termes d'acide, d'un ou plusieurs tensioactifs anioniques sélectionnés dans le groupe consistant en un sel d'acide N-acylamino et un acide carboxylique d'éther alkylique de polyoxyéthylène ou un sel de celui-ci ;
(B) 2 % en masse ou plus et 50 % en masse ou moins d'un ou plusieurs polyols sélectionnés dans le groupe consistant en glycérine, sorbitol, propylène glycol, butylène glycol, dipropylène glycol et polyéthylène glycol présentant un poids moléculaire moyen en masse de 20 000 ou moins ; et
(C) 40 % en masse ou plus et 90 % en masse ou moins d'eau ; dans lequel une teneur totale des composants (A) et (B) dans la composition de nettoyage de la peau est de 10 % en masse ou plus et 60 % en masse ou moins ;
dans lequel le poids moléculaire moyen en masse du polyéthylène glycol est mesuré par chromatographie par perméation sur gel.
